# EUROPEAN PATENT SPECIFICATION

(11) **EP 4 519 281 B1**
(45) Date of publication and mention of the grant of the patent: **28.01.2026**
(21) Application number: 23720834.3
(22) Date of filing: 19.04.2023
(51) Int. Cl.: C07J 9/00, C07J 41/00

(54) **PROCESS FOR THE PREPARATION OF CHOLIC ACID DERIVATIVES**
VERFAHREN ZUR HERSTELLUNG VON CHOLSÄUREDERIVATEN
PROCÉDÉ DE PRÉPARATION DE DÉRIVÉS D'ACIDE CHOLIQUE

(30) Priority: 03.05.2022 IT 202200008861
(43) Date of publication of application: 12.03.2025
(73) Proprietor: CHEMELECTIVA S.R.L., 28100 Novara (IT)
(72) Inventor: CASTALDI, Michele, 21029 Vergiate (VA) (IT); VERZOLETTO, Paolo, 13835 Valdilana (BI) (IT); CASTALDI, Graziano, 28072 Briona (NO) (IT)
(74) Representative: Longoni, Alessandra
(86) International application number: PCT/EP2023/060098
(87) International publication number: WO 2023/213540

(56) References cited:
- WO-A1-2020/231776
- WO-A1-2021/214685
- CZAJKOWSKA ET AL: "Synthesis of cholaphanes by ring closing metathesis", TETRAHEDRON LETTERS, ELSEVIER, AMSTERDAM , NL, vol. 48, no. 16, 22 March 2007 (2007-03-22), pages 2851 - 2855, XP005934060, ISSN: 0040-4039, DOI: 10.1016/J.TETLET.2007.02.099
- PELLICCIARI R ET AL: "BILE ACID DERIVATIVES AS LIGANDS OF THE FARNESOID X RECEPTOR. SYNTHESIS, EVALUATION, AND STRUCTURE-ACTIVITY RELATIONSHIP OF A SERIES OF BODY AND SIDE CHAIN MODIFIED ANALOGUES OF CHENODEOXYCHOLIC ACID", JOURNAL OF MEDICINAL CHEMISTRY, AMERICAN CHEMICAL SOCIETY, US, vol. 47, 26 August 2004 (2004-08-26), pages 4559 - 4569, XP002569577, ISSN: 0022-2623, [retrieved on 20040723], DOI: 10.1021/JM049904B
- G. PIANCATELLI ET AL: "The Synthesis of a C23-Spiroketalic Steroidal Sapogenin", GAZZETTA CHIMICA ITALIANA, vol. 104, 1 January 1974 (1974-01-01), pages 343 - 356, XP009541265

## Description

The present invention relates to a process for the preparation of cholic acid derivatives, in particular of compounds of formula (V):

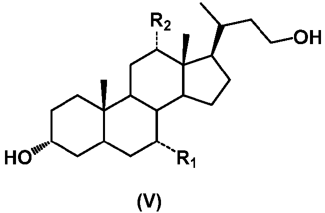

The present invention also relates to compounds useful for the preparation of such derivatives.

Cholic acid is a primary bile acid synthesized in the liver from cholesterol through multiple complementary enzymatic processes. Bile acids include a group of molecular species with similar chemical structures, which are secreted in the bile and transported into the lumen of the small intestine where they act as emulsifiers aiding fat digestion and absorption, as well as endocrine molecules capable of controlling different signaling pathways.

Bile acids (BA) are not only digestive surfactants, but also important cell signaling molecules, which stimulate different signaling pathways to regulate some important biological processes. The nuclear bile acid-activated receptor, the farnesoid X receptor (FXR), plays a critical role in the regulation of bile acid, lipid, and glucose homeostasis, as well as in the regulation of inflammatory responses, barrier function, and the prevention of bacterial translocation in the intestinal tract. As expected, FXR is involved in the pathophysiology of a broad range of diseases of the gastrointestinal tract, including inflammatory bowel disease, colorectal cancer, and type 2 diabetes.

The identification of new steroid molecules capable of binding and modulating the FXR receptor has proven to be of significant importance for the discovery of new possible therapies.

Particularly interesting in this sense were steroid derivatives having one carbon atom less on the side chain, such as BAR502 and nor-UDCA.

Most of the synthetic protocols involving BA therefore include side chain modification and for this purpose the carboxylic functional group placed in C-24 plays a crucial role.

The dehomologation of carboxylic acids allows the adaptation of a carbon chain to the desired length and can be obtained with different methodologies.

There are several general protocols known in the literature for the dehomologation of the carboxylic functional group to the corresponding alcohol, with specific reference to examples of steroid derivatives.

M.F. Saraiva et al., Tetrahedron, 2009, 65, 3563-3572 describes the following process for the transformation of cholic acid derivatives into the corresponding bromides with one carbon atom less by the Barton-McCombie reaction using 2-mercaptopyridine N-oxide sodium salt and bromotrichloromethane.

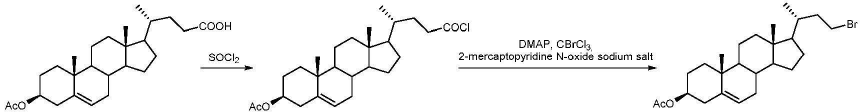

Hernández-Huerta E. et al; "A Straightforward One-Pot Two-Step Conversion of Bile Acids into Dehomologated Alcohols", Steroids 2021, 176, 108917 describes a one-pot procedure which allows the synthesis of dehomologated alcohols from carboxylic acids with the use of hypervalent iodine, MCPBA and photochemical irradiation.

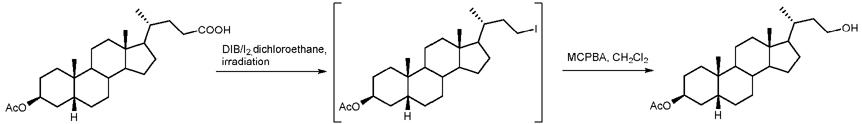

The above examples are not industrially convenient, as they involve complex technologies and isolation and purification procedures.

Another example of an approach reported in the literature, with reference to cholic acid derivatives, consists in the rearrangement of the carboxylic acids into the corresponding dehomologated amines.

Pellicciari R. et al. J. Med. Chem. 2004, 47, 4559-4569 describes a process in which the Schmidt reaction is used. The preparation of the corresponding acyl chloride is followed by the reaction with sodium azide to give the corresponding acyl azide. This, in turn, is rearranged into the corresponding isocyanate which is reacted with an alcohol to give the corresponding carbamate and subsequently hydrolyzed to the final dehomologated amine.

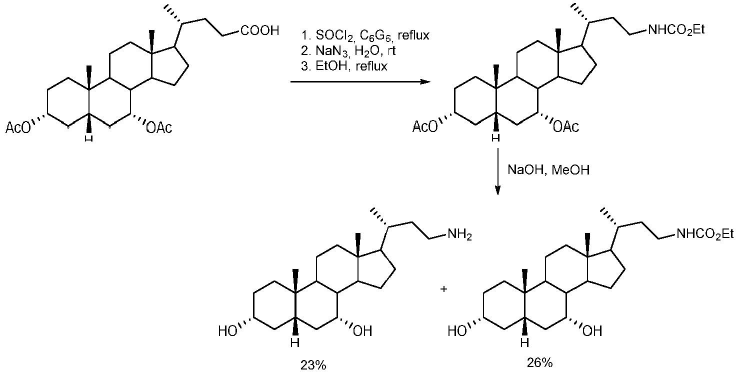

Again, the multi-step procedure is complex as it requires the protection of the hydroxyl groups which are not compatible with the formation of the acyl chloride using thionyl chloride. Furthermore, this procedure leads to mixtures of products making it unsuitable for industrial scalability.

WO-A-2021/214685 and Tet. Lett. Vol 48(16) pp 2861-2855 (2007) disclose processes for the preparation of the same nor-bile alcohol compounds as are prepared according to the process of the present invention, but these publications achieve the reduction in the length of the C17-side chain by cyanation and phenylation/oxidation respectively and not via the introduction of an isocyanate group.

There is therefore the need to find a new synthetic approach to dehomologate the side chain of derived cholic acids which employs simple and repeatable reactions on an industrial scale.

The object of the present invention is therefore a process for the preparation of a compound of formula (V) 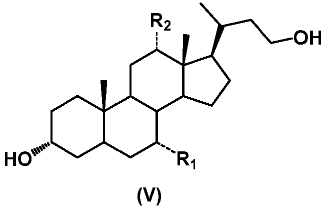 which comprises:
a) Reacting a compound of formula (I) 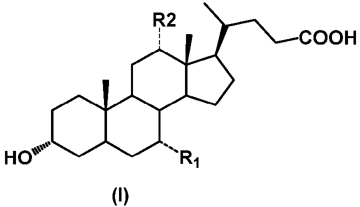
   with an organic azide of formula R-N₃ in an aprotic solvent in the presence of a base wherein R is selected from diphenylphosphoryl, trimethylsilyl, trifluoromethanesulfonyl, preferably a diphenylphosphoryl group.
   to give the corresponding isocyanate of formula (II) 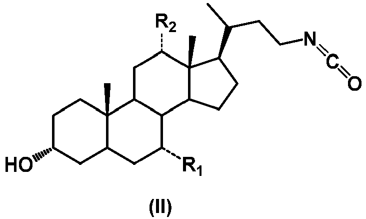 in which
      R₁ represents a -C=O group, or an OH group either in α or β configuration;
      R₂ represents a hydrogen atom, or an OH group;
b) Reacting the isocyanate of formula (II) thus obtained with an alcohol of formula R₃-OH to give the carbamate of formula (III) 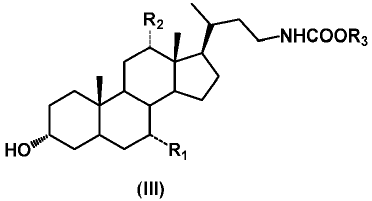 in which
   R₁ and R₂ have the above meanings;
   R₃ represents a linear or branched C₁₋₁₀ alkyl, aryl or alkylaryl.
c) Hydrolyzing the carbamate of formula (III) in the presence of an acid or a base to give the amine of formula (IV) 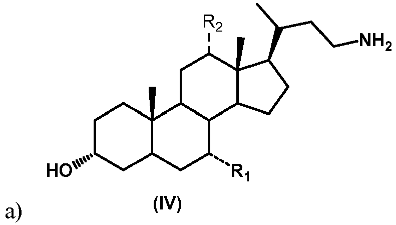 in which R₁ and R₂ have the above meanings.
d) Subjecting the compound of formula (IV) to a diazotization reaction in the presence of a suitable reagent to provide the corresponding desired alcohol of formula (V).

The process of the present invention is a dehomologation process for the preparation of an alcohol derivative of a cholic acid starting from the corresponding carboxylic acid, in which said alcohol derivative contains one carbon atom less than the starting carboxylic acid.

The term "dehomologation" according to the present invention means one or more reactions which allow the side chain of the starting cholic acid derivative to be shortened by a -CH₂ group.

Step a) of the process object of the present invention consists of a Curtius reaction which, as it is well known to the skilled person, is carried out by reacting a carboxylic acid of formula (I) dissolved in an aprotic solvent with an organic azide of formula R-N₃, wherein R is selected from a diphenylphosphoryl, trimethylsilyl, trifluoromethanesulphonyl group, preferably it is a diphenylphosphoryl group.

Examples of aprotic solvents which can be used in step a) are N-methylpyrrolidone, N,N-dimethylacetamide, N,N-dimethylformamide, tetrahydrofuran, methyltetrahydrofuran, toluene, methylene chloride and mixtures thereof, preferably tetrahydrofuran.

To the reaction mixture containing the acid of formula (I) dissolved in the above aprotic solvent and the azide, a tertiary amine selected from triethylamine, tributylamine, diisopropylethylamine, N,N-dimethylaminopyridine is added.

Preferably said tertiary amine is triethylamine.

According to the present invention, said reaction is carried out at a temperature ranging from room temperature to the reflux temperature of the solvent for a time comprised between 2 to 6 hours, preferably about 4 hours.

Once the reaction has been completed, the isocyanate of formula (II) is isolated by techniques well known to those skilled in the art, such as for example filtration, extraction, precipitation, crystallization.

Subsequently, the isocyanate of formula (II) thus obtained is subjected in step b) of the present process to a reaction with an alcohol of formula R₃-OH, wherein R₃ is a linear or branched alkyl C₁-C₄ group, an aryl or an alkylaryl.

Said alcohol is preferably selected from methanol, ethanol, isopropanol, tert-butanol, more preferably it is tert-butanol.

As it will be clear to the person skilled in the art, steps a) and b) can be carried out as described above, i.e. by isolating the isocyanate of formula (II) formed at the end of step a).

Alternatively, steps a) and b) can be performed "one pot", i.e. without isolating the intermediate compound.

Preferably, steps a) and b) are carried out "one pot", i.e. with the use of R₃-OH directly as the reaction solvent.

According to the present invention, the carbamate of formula (III) is hydrolyzed in step c) of the process in the presence of an acid preferably selected from hydrochloric acid, sulfuric acid, or in the presence of a base preferably selected from sodium hydroxide, potassium hydroxide, sodium carbonate, potassium carbonate, more preferably potassium hydroxide.

Preferably, said acid or said base is used in a molar amount comprised between 0.8 equivalents and 5 equivalents, more preferably comprised between 1 equivalent and 2.5 equivalents, with respect to the molar amount of carbamate of formula (III).

This hydrolysis reaction takes place in a polar protic solvent selected from methanol, ethanol, isopropanol, 1-methoxy-2-propanol, butanol, preferably 1-methoxy-2-propanol.

Contrary to the previous synthetic methods described in the art, thanks to the process of the present invention, it was possible to isolate the amine of formula (IV) with a yield of 87% and a conversion of 95%.

Once the amine of formula (IV) has been isolated by techniques well known to the skilled person, according to the present invention, said amine is subjected to a diazotization reaction in step d).

This reaction is carried out in the presence of a suitable reagent capable of releasing a nitrosonium ion, such as for example sodium nitrite, in acetic acid alone or in a mixture with water, preferably in acetic acid alone, or an organic nitrite R-NO₂, where R is a linear or branched alkyl group.

Preferably, sodium nitrite is used in a molar amount comprised between 1 equivalent and 3 equivalents, more preferably of about 2 equivalents, with respect to the molar amount of amine of formula (IV).

According to a particularly preferred embodiment, the process of the present invention allows to dehomologate the side chain of a cholic acid derivative, such as 7K-LCA (compound of formula (la)), for the preparation of a key intermediate of the BAR502, or the compound of formula (Va): 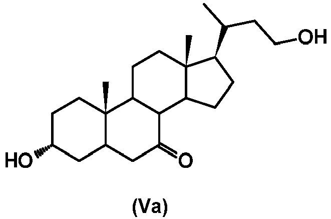 or of the corresponding acetyl derivative of formula (Vb), in turn hydrolyzed to give the compound of formula (Va).

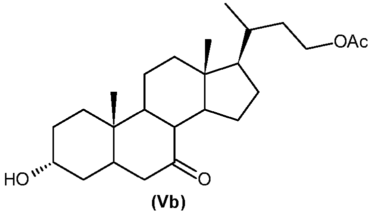

Therefore, the present invention is preferably directed to a process for the preparation of a compound of formula (Va) 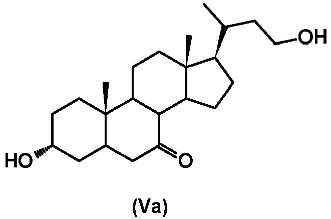 which comprises:
a) Reacting a compound of formula (Ia) 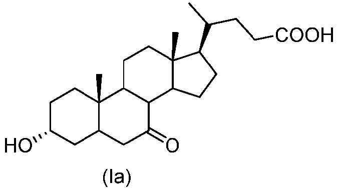 with an organic azide of formula R-N₃ in an aprotic solvent in the presence of a base wherein R is selected from diphenylphosphoryl, trimethylsilyl, trifluoromethanesulfonyl, to give the corresponding isocyanate of formula (IIa) 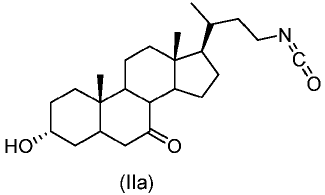
b) Reacting the isocyanate of formula (IIa) thus obtained with R₃-OH to give the carbamate of formula (III) 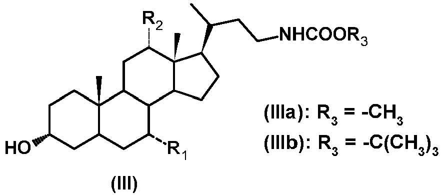 wherein R₃ is selected from methyl and tert-butyl,
c) Hydrolyzing the carbamate of formula (Illa) or (IIIb) in the presence of a base to give the amine of formula (IVa) 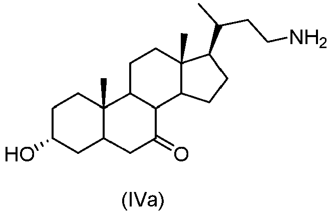 **In which** R₁ and R₂ have the above meanings.
d) Subjecting the compound of formula (IVa) to a diazotization reaction in the presence of sodium nitrite in acetic acid to give the corresponding desired alcohol of formula (Va).

As can be seen, the compounds of formula (la), (IIa), (IIIa), (lllb), (IVa) and (Va) correspond to the compounds of formula (I), (II), (III), (IV) and (V) respectively in which the meanings of R₁, R₂ and R₃ are as follows:
- R₁ represents a group -C=O;
- R₂ represents a hydrogen atom;
- R₃ represents a methyl or tert-butyl group.

The compounds of formula (IIa), (Illa), (IIIb) and (IVa) are new and constitute a further object of the present invention.

Furthermore, these compounds are useful intermediates for the synthesis of cholic acid derivatives, in particular of a key intermediate for the synthesis of BAR502, i.e. the compound of formula (Va).

A further object is therefore the use of the compounds of formula (IIa), (Illa), (IIIb), (IVa) as intermediates in the synthesis of cholic acid derivatives and preferably in the synthesis of the compound of formula (Va).

The present invention will now be illustrated by means of some examples, which are not to be seen as limiting the scope of the invention.

### EXAMPLES

### EXAMPLE 1. Synthesis of methyl ((3R)-3-((3R,10S,13R,17R)-3-hydroxy-10,13-dimethyl-7-oxohexadecahydro-1H-cyclopenta[a]phenanthren-17-yl) butyl)carbamate.

7-keto lithocholic acid (20.0 g, 1 eq), tetrahydrofuran (100.0 mL, 5 vol) was charged to a reaction flask, the temperature was brought to about 25°C, and triethylamine (8.5 ml, 1.2 eq) and diphenylphosphorylazide (DPPA, 15.5 g, 1.1 eq) were added. The reaction was heated to reflux and kept under these conditions for about 2 hours. The temperature was brought to room temperature and the resulting solution was dropped for about 4 hours in a 30% solution of sodium methoxide in methanol (48.3 mL, 5 eq). The reaction mixture was kept under these conditions for about two hours and, when the reaction was finished, methyl tetrahydrofuran (200.0 ml, 10 vol) and water (100.0 ml, 5 vol) were added. The organic phase was washed with 1M sodium hydroxide (2 x 200.0 mL), dried with magnesium sulfate and reduced to a residue by distillation under reduced pressure to give 20.5 g of methyl ((3R)-3-((3R, 10S,13R, 17R)-3-hydroxy-10,13-dimethyl-7-oxohexadecahydro-1H-cyclopenta[a]phenanthren-17-yl)butyl)carbamate (96%).

¹H-NMR (d6-DMSO, 400MHz): δ 6.98 (t, 1H), δ 4.5 (s, 1H), δ 3.5 (s, 3H), δ 3.35 (m, 1H), δ 3.03 (m, 1H), δ 2.9 (m, 2H), δ 2.46 (t, 1H), δ 2.07 (m, 1H), δ 1.8 (d, 1H), δ 1.7 (m, 5H), δ 1.5 (m, 3H), δ 1.4 (m, 4H), δ 1.13 (m, 10H), δ 0.9 (d, 4H), δ 0.62 (s, 3H).

¹³C-NMR (d6-DMSO, 400MHz): δ 211.6, δ 157.03, δ 69.6, δ 55.02, δ 51.5, δ 49.3, δ 49.05, δ 42.65, δ 39.1, δ 38.26, δ 37.85, δ 36.16, δ 35.2, δ 34.36, δ 33.48, δ 30.27, δ 28.41, δ 24.89, δ 23.23, δ 21.71, δ 21.32, δ 19.01, δ 12.28.

### EXAMPLE 2. Synthesis of (3R,10S,13R,17R)-17-((R)-4-aminobutan-2-yl)-3-hydroxy-10,13-dimethylhexadecahydro-7H-cyclopenta[a]phenanthrene-7-one.

In a reaction flask, methyl-((3R)-3-((3R,10S,13R,17R)-3-hydroxy-10,13-dimethyl-7-oxohexadecahydro-1H-cyclopenta[a]phenanthrene- 17-yl)butyl)carbamate (10.0 g, 1 eq), 1-methoxy-2-propanol (50.0 ml, 5 vol) and potassium hydroxide (3.0 g, 2 eq) were charged and the temperature was brought to about 25°C. The resulting suspension was heated to reflux and kept under these conditions for about 16 hours. At the end of the reaction, the mixture was brought to room temperature and filtered on paper. The residual solid was washed with 1-methoxy-2-propanol (2 x 25.0 mL) and concentrated by distillation under reduced pressure to give 7.5 g of (3R,10S,13R,17R)-17-((R)-4-aminobutan-2-yl)-3-hydroxy-10,13-dimethylhexadecahydro-7H-cyclopenta[a]phenanthren-7-one (87%).

¹H-NMR (d6-DMSO, 400MHz): δ 3.35 (m, 1H), δ 2.89 (m, 1H), δ 2.5 (s, 2H), δ 2.44 (m, 2H), δ 2.05 (m, 1H), δ 1.93 (d, 1H), δ 1.8 (m, 3H), δ 1.4 (m, 5H), δ 1.09 (s, 3H), δ 1.07 (m, 4H), δ 1.02 (d, 2H), δ 0.88 (d, 4H), δ 0.62(s, 3H).

¹³C-NMR (d6-DMSO, 400MHz): δ 211.85, δ 129.2, δ 69.6, δ 55.36, δ 49.3, δ 49.05, δ 42.65, δ 37.9, δ 35.2, δ 34.36, δ 33.6, δ 30.3, δ 28.55, δ 24.89, δ 23.26, δ 21.69, δ 20.75, δ 19.3, δ 12.3

### EXAMPLE 3. Synthesis of tert-butyl ((3R)-3-((3R,10S,13R,17R)-3-hydroxy-10,13-dimethyl-7-oxohexadecahydro-1H-cyclopenta[a]phenanthrene-17-yl)butyl)carbamate.

7-keto lithocholic acid (10.0 g, 1 eq), tert-butanol (50.0 ml, 5 vol) and tetrahydrofuran (10.0 ml, 1 vol) were charged to a reaction flask to give a colorless solution.

Triethylamine (4.3 ml, 1.2 eq) and diphenylphosphorylazide (DPPA, 7.75 g, 1.1 eq) were added to the solution and the mixture was heated to reflux and kept under these conditions for about 4 hours. At the end of the reaction, the temperature was brought to about 20°C and methyl tetrahydrofuran (100.0 ml, 10 vol) and a 2M sodium hydroxide solution (30.0 ml, 3 vol) were added. The organic phase was washed with 2M sodium hydroxide (2 x 50.0 mL) and brine (50.0 mL), dried with magnesium sulfate and reduced to a residue by distillation under reduced pressure to give 16.0 g of tert-butyl ((3R)-3-((3R,10S,13R,17R)-3-hydroxy-10,13-dimethyl-7-oxohexadecahydro-1H-cyclopenta[a]phenanthren-17-yl)butyl)carbamate (100%).

¹H-NMR (d6-DMSO, 400MHz): δ 5.7 (t, 1H), δ 4.5 (s, 1H), δ 3.5 (s, 3H), δ 3.03 (m, 1H), δ 2.9 (m, 2H), δ 2.44 (t, 1H), δ 2.07 (m, 1H), δ 1.9 (d, 1H), δ 1.7 (m, 3H), δ 1.5 (m, 3H), δ 1.4 (m, 7H), δ 1.13 (m, 8H), δ 0.9 (d, 4H), δ 0.62(s, 3H)

¹³C-NMR (d6-DMSO, 400MHz): δ 211.6, δ 158.6, δ 139.6, δ 69.6, δ 55.4, δ 55.2, δ 49.3, δ 49.05, δ 45.85, δ 45.53, δ 42.65, δ 42.64, δ 39.1, δ 37.9, δ 37.21, δ 36.7, δ 35.2, δ 34.85, δ 34.36, δ 33.48, δ 30.89, δ 30.27, δ 28.7, δ 28.41, δ 24.89, δ 23.23, δ 21.71, δ 19.11, δ 12.37

### EXAMPLE 4. Synthesis of (3R,10S,13R,17R)-17-((R)-4-aminobutan-2-yl)-3-hydroxy-10,13-dimethylhexadecahydro-7H-cyclopenta[a]phenanthrene-7- one.

Tert-butyl ((3R)-3-((3R,10S,13R,17R)-3-hydroxy-10,13-dimethyl-7-oxohexadecahydro-1H-cyclopenta[a]phenanthren-17-yl)butyl)carbamate (14.0 g, 1eq), acetonitrile (70.0 ml, 5 vol) and methanol (70.0 ml, 5 vol) were charged into a reaction flask and the temperature was brought to about 25°C. A 37% hydrochloric acid solution (4.0 mL, 1.5 eq) was dropped over 10 minutes into the reaction. The reaction was maintained under these conditions for about 6 hours. When the reaction was complete, the solvent was removed and methyl tetrahydrofuran (210.0 mL, 15 vol) and tetrahydrofuran (70.0 mL, 5 vol) were added. The mixture was washed with hydrochloric acid (280.0 mL) and filtered through celite washing with methyl tetrahydrofuran (70.0 mL, 5 vol) and tetrahydrofuran (28.0 mL, 2 vol). The aqueous phase was basified at pH = 10 with sodium carbonate. Methyl tetrahydrofuran (140.0 mL, 15 vol) and tetrahydrofuran (52.0 mL, 5 vol) were added. The organic phase was then dried with magnesium sulfate, filtered and reduced to a residue by distillation under reduced pressure to give 9.3 g of (3R,10S,13R,17R)-17-((R)-4-aminobutan-2-yl)-3-hydroxy-10,13-dimethylhexadecahydro-7H-cyclopenta[a]phenanthrene-7-one (80%).

¹H-NMR (d6-DMSO, 400MHz): δ 3.35 (m, 1H), δ 2.89 (m, 1H), δ 2.5 (s, 2H), δ 2.44 (m, 2H), δ 2.05 (m, 1H), δ 1.93 (d, 1H), δ 1.8 (m, 3H), δ 1.4 (m, 5H), δ 1.09 (s, 3H), δ 1.07 (m, 4H), δ 1.02 (d, 2H), δ 0.88 (d, 4H), δ 0.62(s, 3H).

¹³C-NMR (d6-DMSO, 400MHz): δ 211.85, δ 129.2, δ 69.6, δ 55.36, δ 49.3, δ 49.05, δ 42.65, δ 37.9, δ 35.2, δ 34.36, δ 33.6, δ 30.3, δ 28.55, δ 24.89, δ 23.26, δ 21.69, δ 20.75, δ 19.3, δ 12.3

### EXAMPLE 5. Synthesis of (3R,10S,13R,17R)-3-hydroxy-17-((R)-4-hydroxybutan-2-yl)-10,13-dimethylhexadecahydro-7H-cyclopenta[a]phenanthren-7 -one.

(3R,10S,13R,17R)-17-((R)-4-aminobutan-2-yl)-3-hydroxy-10,13-dimethylhexadecahydro-7H-cyclopenta[a]phenanthrene-7-one (1.0 g, 1 eq) and acetic acid (5.0 ml, 5 vol) were charged into a reaction flask, the reaction temperature was brought to about 25°C and sodium nitrite was added in small portions (0.38 g, 2 eq). The mixture was kept under stirring under these conditions for about 16 hours. At the end of the reaction, this mixture was brought to pH = 9 by adding sodium bicarbonate. The product was extracted with ethyl acetate (10.0 mL, 10 vol) and the organic phase was washed with an aqueous solution of sodium bicarbonate (55.0 mL, 5 vol) and brine (5.0 mL, 5 vol). The solvent was removed by vacuum distillation to give an oily mixture containing the acetylated derivative. The mixture was subsequently treated with a solution of 30% sodium hydroxide (5 eq) in methanol (10 vol) at reflux until complete conversion after 16 hours to give (3R,10S,13R,17R)-3-hydroxy-17 -((R)-4-hydroxybutan-2-yl)-10,13-dimethylhexadecahydro-7H-cyclopenta[a]phenanthren-7-one (80%) and an analytically pure sample was purified by column chromatography (eluent dichloromethane :methanol 98:2).

¹H-NMR (d6-DMSO, 400MHz): δ 4.49 (m, 1H), δ 4.25 (m, 1H), δ 3.45 (s, 1H), δ 3.35 (s 4H), δ 2.9 (m, 1H), δ 2.44 (t, 1H), δ 2.1 (m, 1H), δ 1.9 (m, 1H), δ 1.8 (m, 2H), δ 1.7 (m, 3H), δ 1.14 (s, 3H), δ 1.08 (m, 5H), δ 0.9 (d, 4H), δ 0.62 (s, 3H).

¹³C-NMR (d6-DMSO, 400MHz): δ 211.85, δ 69.6, δ 58.9, δ 55.4, δ 49.3, δ 49.05, δ 45.85, δ 45.5, δ 42.7, δ 42.65, δ 37.9, δ 35.2, δ 34.36, δ 30.3, δ 28.55, δ 24.89, δ 23.26, δ 21.69, δ 19.3, δ 12.3

## Claims

1. Process for the preparation of a compound of formula (V) 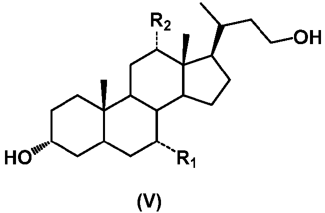 comprising:
a) Reacting a compound of formula (I) 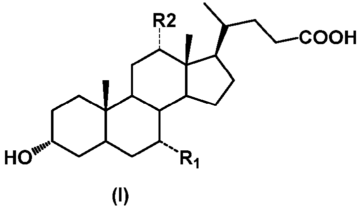
with an organic azide of formula R-N₃ in an aprotic solvent in the presence of a base in which R is selected from diphenylphosphoryl, trimethylsilyl, trifluoromethanesulfonyl
to give the corresponding isocyanate of formula (II) 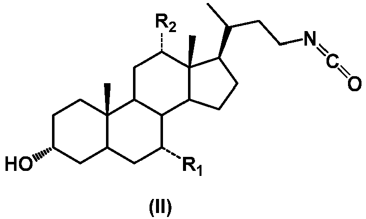 in which
R₁ represents a -C=O group, or an OH group either in α or β configuration;
R₂ represents a hydrogen atom, or an OH group;
b) Reacting the isocyanate of formula (II) thus obtained with an alcohol of formula R₃-OH to provide the carbamate of formula (III) 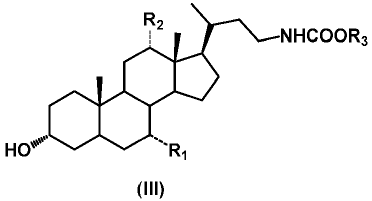 in which
R₁ e R₂ have the above-mentioned meanings;
R₃ represents a C₁₋₁₀ linear or branched alkyl group, an aryl or alkylaryl group.
c) Hydrolyzing the carbamate of formula (III) in the presence of an acid or a base to give the amine of formula (IV) 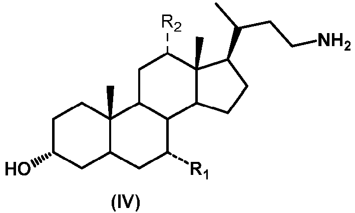 in which R₁ e R₂ have the above-mentioned meanings.
d) Subjecting the compound of formula (IV) to a diazotation reaction in the presence of an adequate reagent to give the desired alcohol of formula (V).

2. Process according to claim 1, wherein said azide of formula R-N₃ in step a) is diphenylphosphoryl azide.

3. Process according to any one of the preceding claims, wherein said base in step a) is a tertiary amine, preferably selected from triethylamine, tributylamine, diisopropylethylamine, N,N-dimethylaminopyridine.

4. Process according to any one of the preceding claims, wherein said alcohol of formula R₃-OH in step b) is selected from methanol, ethanol, isopropanol, tert-butanol, preferably is tert-butanol.

5. Process according to any one of the preceding claims, wherein steps a) and b) are performed "one pot".

6. Process according to any one of the preceding claims, wherein said acid or said base in step c) is used in a molar quantity comprised between 0.8 equivalents and 5 equivalents, preferably comprised between 1 equivalent and 2.5 equivalents, with respect to the molar quantity of the carbamate of formula (III).

7. Process according to any one of the preceding claims, wherein said adequate reagent in step d) is sodium nitrite.

8. Process according to claim 7, wherein said sodium nitrite is used in a molar quantity comprised between 1 equivalent and 3 equivalents, preferably of about 2 equivalents, with respect to the molar quantity of the amine of formula (IV).

9. Process according to any one of the preceding claims, in which:
- R₁ represents a -C=O group;
- R₂ represents a hydrogen atom;
- R₃ represents a methyl or a tert-butyl group.

10. Use of the compounds of formula: 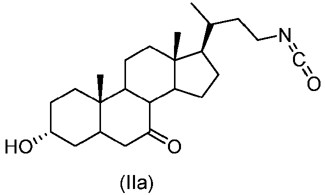 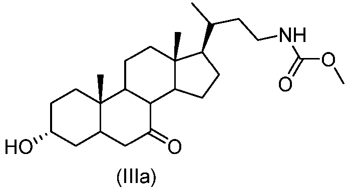 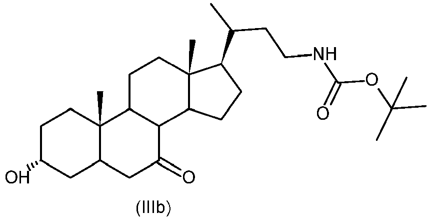 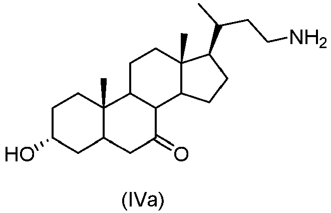 as intermediates in the process according to any one of claims 1-9.

## Patentansprüche

1. Verfahren zur Herstellung einer Verbindung der Formel (V) 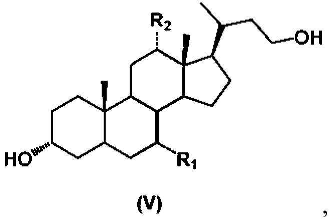 umfassend:
a) Umsetzen einer Verbindung der Formel (I) 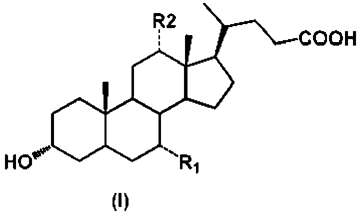
mit einem organischen Azid der Formel R-N₃ in einem aprotischen Lösungsmittel in der Gegenwart einer Base, in der R ausgewählt ist aus Diphenylphosphoryl, Trimethylsilyl, Trifluormethansulfonyl,
um das entsprechende Isocyanat der Formel (II) zu ergeben 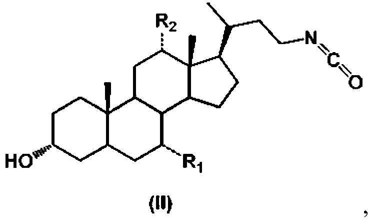 in dem
R₁ eine -C=O-Gruppe oder eine OH-Gruppe entweder in α- oder β-Konfiguration darstellt;
R₂ ein Wasserstoffatom oder eine OH-Gruppe darstellt;
b) Umsetzen des so erlangten Isocyanats der Formel (II) mit einem Alkohol der Formel R₃-OH, um das Carbamat der Formel (III) bereitzustellen 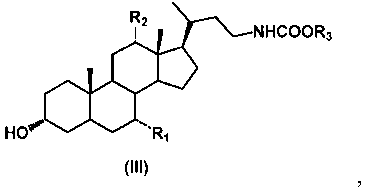 in dem
R₁ und R₂ die vorstehend erwähnten Bedeutungen aufweisen;
R₃ eine lineare oder verzweigte C₁₋₁₀-Alkylgruppe, eine Aryl- oder Alkylarylgruppe darstellt,
c) Hydrolysieren des Carbamats der Formel (III) in der Gegenwart einer Säure oder einer Base, um das Amin der Formel (IV) zu ergeben 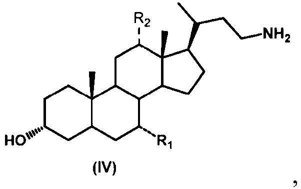 in dem R₁ und R₂ die vorstehend erwähnten Bedeutungen aufweisen,
d) Unterziehen der Verbindung der Formel (IV) einer Diazotierungsreaktion in der Gegenwart eines zweckmäßigen Reagens, um den gewünschten Alkohol der Formel (V) zu ergeben.

2. Verfahren nach Anspruch 1, wobei das Azid der Formel R-N₃ in Schritt a) Diphenylphosphorylazid ist.

3. Verfahren nach einem der vorhergehenden Ansprüche, wobei die Base in Schritt a) ein tertiäres Amin ist, bevorzugt ausgewählt aus Triethylamin, Tributylamin, Diisopropylethylamin, N,N-Dimethylaminopyridin.

4. Verfahren nach einem der vorhergehenden Ansprüche, wobei der Alkohol der Formel R₃-OH in Schritt b) ausgewählt ist aus Methanol, Ethanol, Isopropanol, tert-Butanol, bevorzugt tert-Butanol ist.

5. Verfahren nach einem der vorhergehenden Ansprüche, wobei Schritt a) und b) "im Ein-Topf-Verfahren" durchgeführt werden.

6. Verfahren nach einem der vorhergehenden Ansprüche, wobei die Säure oder die Base in Schritt c) in einer molaren Menge, die zwischen 0,8 Äquivalenten und 5 Äquivalenten umfasst ist, bevorzugt zwischen 1 Äquivalent und 2,5 Äquivalenten umfasst ist, in Bezug auf die molare Menge des Carbamats der Formel (III) verwendet wird.

7. Verfahren nach einem der vorhergehenden Ansprüche, wobei das zweckmäßige Reagens in Schritt d) Natriumnitrit ist.

8. Verfahren nach Anspruch 7, wobei das Natriumnitrit in einer molaren Menge, die zwischen 1 Äquivalent und 3 Äquivalenten umfasst ist, bevorzugt von etwa 2 Äquivalenten, in Bezug auf die molare Menge des Amins der Formel (IV) verwendet wird.

9. Verfahren nach einem der vorhergehenden Ansprüche, in dem:
- R₁ eine -C=O-Gruppe darstellt;
- R₂ ein Wasserstoffatom darstellt;
- R₃ eine Methyl- oder eine tert-Butylgruppe darstellt.

10. Verwendung der Verbindungen der Formel: 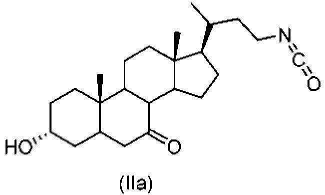 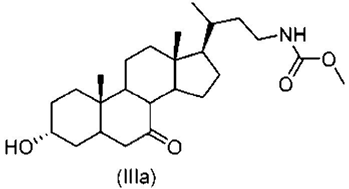 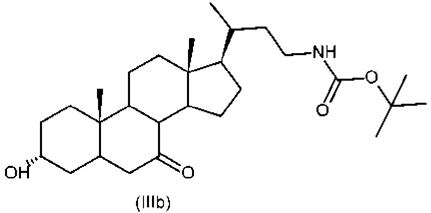 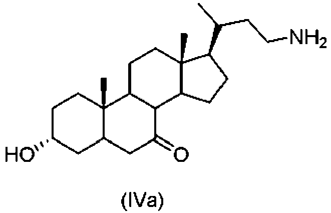 als Zwischenprodukte in dem Verfahren nach einem der Ansprüche 1-9.

## Revendications

1. Processus pour la préparation d'un composé de formule (V) 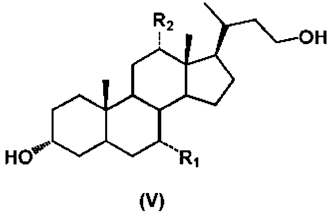 comprenant :
a) la réaction d'un composé de formule (I) 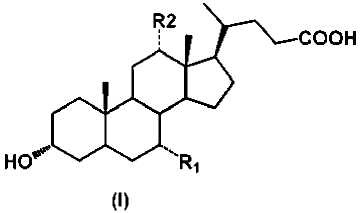 avec un azoture organique de formule R-N₃ dans un solvant aprotique en présence d'une base, où R est choisi parmi un diphénylphosphoryle, un triméthylsilyle, un trifluorométhanesulfonyle pour donner l'isocyanate correspondant de formule (II) 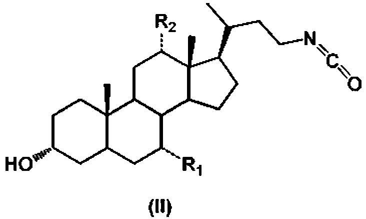 où
R₁ représente un groupe -C=O, ou un groupe OH en configuration α ou β ;
R₂ représente un atome d'hydrogène ou un groupe OH ;
b) la réaction de l'isocyanate de formule (II) ainsi obtenu avec un alcool de formule R₃-OH pour fournir le carbamate de formule (III) 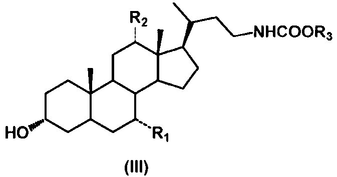 où
R₁ et R₂ ont les significations susmentionnées ;
R₃ représente un groupe alkyle linéaire ou ramifié en C₁₋₁₀, un groupe aryle ou alkylaryle,
c) l'hydrolyse du carbamate de formule (III) en présence d'un acide ou d'une base pour donner l'amine de formule (IV) 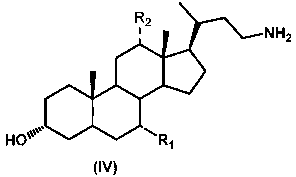 où R₁ et R₂ ont les significations susmentionnées,
d) la soumission du composé de formule (IV) à une réaction de diazotation en présence d'un réactif adéquat pour donner l'alcool souhaité de formule (V).

2. Processus selon la revendication 1, dans lequel ledit azoture de formule R-N₃ de l'étape a) est l'azoture de diphénylphosphoryle.

3. Processus selon l'une quelconque des revendications précédentes, dans lequel ladite base de l'étape a) est une amine tertiaire, de préférence choisie parmi la triéthylamine, la tributylamine, la diisopropyléthylamine, la N,N-diméthylaminopyridine.

4. Processus selon l'une quelconque des revendications précédentes, dans lequel ledit alcool de formule R₃-OH de l'étape b) est choisi parmi le méthanol, l'éthanol, l'isopropanol, le tert-butanol, de préférence le tert-butanol.

5. Processus selon l'une quelconque des revendications précédentes, dans lequel les étapes a) et b) sont effectuées « de manière monotope ».

6. Processus selon l'une quelconque des revendications précédentes, dans lequel ledit acide ou ladite base de l'étape c) est utilisé(e) en quantité molaire comprise entre 0,8 équivalent et 5 équivalents, de préférence comprise entre 1 équivalent et 2,5 équivalents, par rapport à la quantité molaire du carbamate de formule (III).

7. Processus selon l'une quelconque des revendications précédentes, dans lequel ledit réactif adéquat de l'étape d) est le nitrite de sodium.

8. Processus selon la revendication 7, dans lequel ledit nitrite de sodium est utilisé en une quantité molaire comprise entre 1 équivalent et 3 équivalents, de préférence d'environ 2 équivalents, par rapport à la quantité molaire de l'amine de formule (IV).

9. Processus selon l'une quelconque des revendications précédentes, dans lequel :
- R₁ représente un groupe -C=O ;
- R₂ représente un atome d'hydrogène ;
- R₃ représentant un groupe méthyle ou tert-butyle.

10. Utilisation des composés de formule : 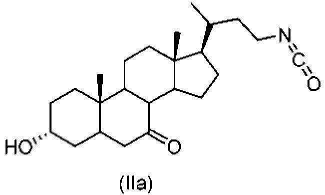 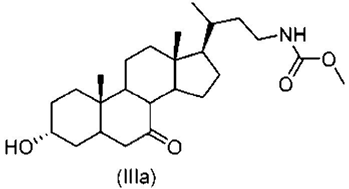 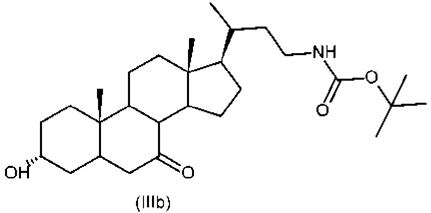 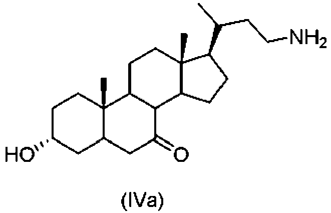 comme intermédiaires dans le processus selon l'une quelconque des revendications 1 à 9.
